# EUROPEAN PATENT SPECIFICATION

(11) **EP 3 364 887 B1**
(45) Date of publication and mention of the grant of the patent: **03.12.2025**
(21) Application number: 16791188.2
(22) Date of filing: 20.10.2016
(51) Int. Cl.: A61B 17/15, A61B 17/17, A61B 17/88

(54) **TWO-PART SURGICAL GUIDE**
ZWEITEILIGE CHIRURGISCHE FÜHRUNG
GUIDE CHIRURGICAL EN DEUX PARTIES

(30) Priority: 22.10.2015 US 201562245255 P
(43) Date of publication of application: 29.08.2018
(73) Proprietor: Materialise NV, 3001 Leuven (BE)
(72) Inventor: MARIEN, Rosalien, 3001 Leuven (BE); LENAERTS, Toon, 3001 Leuven (BE)
(74) Representative: EIP
(86) International application number: PCT/US2016/057867
(87) International publication number: WO 2017/070318

(56) References cited:
- AU-A1- 2014 262 239
- US-A1- 2003 040 748
- US-A1- 2011 015 636
- US-A1- 2011 172 672

## Description

### BACKGROUND OF THE INVENTION

### Field of the Invention

This application relates to surgical guides. More particularly, this application relates to a method of designing a patient-specific system for performing an osteotomy on a bone of a patient.

### Description of the Related Technology

Performing surgical procedures, such as osteotomies (e.g. high-tibia osteotomy (HTO), femoral osteotomies, innominate osteotomies, other tibial osteotomies, etc.) can be a challenging surgical procedure. For example, in an osteotomy, a bone is cut to either shorten, lengthen or realign at least a portion of the bone, such as to change an alignment angle of the bone. In an osteotomy, accurate positioning of the cut in the bone, as well as fixation of the bone fragments in the accurate changed position is important to ensure a successful surgery.

Traditionally, these types of surgeries are planned using X-ray images of the surgical site to determine how best to carry out the procedure. X-ray imaging, however, gives a limited view of the actual three-dimensional ("3-D") anatomy of the patient and on the 3-D correction to be made to the anatomy of the patient (e.g. cut and change in position of the bone of the patient). These limitations in X-ray technology result in the need for extensive checking of various parameters (e.g. cut positions, bone positions, drill positions, alignments, etc.) during surgical procedures. Often, the checking process involves extensive fluoroscopy, which results in a more time-consuming and complicated procedure.

Accordingly, more precise imaging technologies have emerged as improvements for the planning of surgical procedures, such as osteotomies. These more precise imaging technologies, such as computerized tomography (CT) scanning, magnetic resonance imaging (MRI), and the like, allow for precise measurements of the surgical site to be taken prior to the procedure. Accordingly, planned operations, such as planned cuts, drilling or bone fragment repositioning can be precisely mapped out in advance. US Patent Application US2011/015636A1 discloses a patient-specific alignment guide includes a three-dimensional engagement surface customized in a pre-operating planning stage by computer imaging to closely mate and conform to a corresponding bone portion of a patient's elbow joint. The patient-specific alignment guide defines a first longitudinal guiding bore aligned with a reference axis associated with the elbow joint of patient when the alignment guide is mounted onto the corresponding bone portion.

In some cases, patient-specific devices such as patient-specific surgical guides are designed and manufactured based on imaging of the surgical site. However, current patient-specific guides, such as those utilized in osteotomies, have certain drawbacks. For example, these patient-specific guides can be quite bulky, and therefore require larger incisions to be made to utilize the patient-specific guide during the surgical procedure to accommodate the bulky patient-specific guide. For example, large portions of soft-tissue of the patient may need to be cut or moved to accommodate the bulky patient-specific guide. In particular, the current patient-specific guides may need to be designed to interact with enough portions of the bone of the patient to stay in place during surgery, and include all the necessary functional elements (e.g. drill guides, cutting guides, etc.) for guiding the surgeon to ensure the planned operations to the bone are made in the right positions. Further, the current bulky patient-specific guides can make the surgical procedure more challenging and less precise, because the surgical site becomes too crowded. Accordingly, improved patient-specific surgical guides, such as patient-specific osteotomy guides, and techniques for using those patient-specific surgical guides are needed which do not suffer from the drawbacks present in current devices.

### SUMMARY

The appended claims define the present invention, including a method of designing a patient-specific system for performing an osteotomy on a bone of a patient, and related methods and systems - examples are described herein. Other examples are described in the present disclosure which do not fall within the scope of the claims.

The disclosure describes a patient-specific system for performing an osteotomy on a bone of a patient, the system comprising: a first part comprising: at least one contact surface configured to conform to at least one portion of the bone such that when the at least one contact surface is positioned on the at least one portion of the bone, movement of the first part is substantially restricted with respect to the bone; and a plurality of guiding elements configured to guide placement of a plurality of reference pins in the bone; and a second part, separate from the first part, the second part comprising: a plurality of apertures each with a shape corresponding to a reference pin, each of the plurality of apertures being configured to receive one of the plurality of reference pins and substantially restrict movement of the second part with respect to the bone when the plurality of reference pins are received; and at least one functional element for drilling a hole in the bone.

Also described herein although not falling within the scope of the claims is a method for performing an osteotomy on a bone of a patient, the method comprising: placing a first part of a two-part osteotomy guide within a surgical site of the bone of the patient, the first part comprising: at least one contact surface configured to conform to at least one portion of the bone such that when the at least one contact surface is positioned on the at least one portion of the bone, movement of the first part is substantially restricted with respect to the bone; and a plurality of guiding elements configured to guide placement of the reference pins in the bone; positioning the first part on the bone by aligning the at least one contact surface with the at least one portion of the bone to secure the first part to the bone; guiding placement of the reference pins into the bone based on the plurality of guiding elements; removing the first part from the bone; positioning a second part of the two-part osteotomy guide on the bone by receiving the reference pins in a plurality of apertures of the second part, the second part comprising: the plurality of apertures each with a shape corresponding to a reference pin, each of the plurality of apertures being configured to receive one of the plurality of reference pins and substantially restrict movement of the second part with respect to the bone when the plurality of reference pins are received; and at least one functional element for guiding a procedure on the bone; drilling at least one additional hole into the bone through the at least one functional element; removing the second part from the bone; performing an osteotomy cut to the bone; and securing an osteosynthesis implant to the bone using the at least one drilled hole.

Certain embodiments of this disclosure provide a patient-specific system for performing an surgery on a patient, the system comprising: a first part comprising: at least one contact surface configured to conform to at least one portion of an anatomy of the patient such that when the at least one contact surface is positioned on the at least one portion of the anatomy of the patient, movement of the first part is substantially restricted with respect to the anatomy of the patient; and a plurality of guiding elements configured to receive a plurality of reference pins and guide placement of the reference pins in the anatomy of the patient; and a second part, separate from the first part, the second part comprising: a plurality of apertures each with a shape corresponding to a reference pin, each of the plurality of apertures being configured to receive one of the plurality of reference pins and substantially restrict movement of the second part with respect to the anatomy of the patient when the plurality of reference pins are received; and at least one functional element for guiding a procedure on the anatomy of the patient.

### BRIEF DESCRIPTION OF THE DRAWINGS

Figure 1A illustrates 3-D models of a bone of a patient that is to undergo surgery, in accordance with certain embodiments.
Figure 1B is a close-in view of the planned osteotomy cut shown in Figure 1A, in accordance with certain embodiments.
Figure 2 is an example of an existing bulky single piece osteotomy guide having a large footprint and high profile.
Figure 3 provides a front, a side, a top, and a back side view of a first guide in a two-part surgical guide, in accordance with certain embodiments.
Figure 4 provides a front, a side, a top, and a back side view of a second guide in a two-part surgical guide, in accordance with certain embodiments.
Figure 5 is a flow chart illustrating a high-level process for performing an osteotomy using a two-part osteotomy guide, in accordance with certain embodiments.
Figure 6 is an example of a system for designing and manufacturing 3D objects
Figure 7 illustrates a functional block diagram of one example of the computer shown in Figure 6.
Figure 8 shows a high-level process for manufacturing a 3D object using an additive manufacturing system.

### DETAILED DESCRIPTION OF CERTAIN INVENTIVE EMBODIMENTS

The following description and the accompanying figures are directed to certain specific embodiments. The embodiments described in any particular context are not intended to limit this disclosure to the specified embodiment or to any particular usage. Those of skill in the art will recognize that the disclosed embodiments, aspects, and/or features are not limited to any particular embodiments. In certain embodiments and aspects described herein, a surgical procedure can be an osteotomy, an ostectomy, an arthrodesis, an arthroplasty, a chondroplasty, a fracture repair, etc. In certain embodiments and aspects described herein, a bone anatomy can be a tibia, a femur, a fibula, a radius, an ulna, a humerus, a tarsal, a metatarsal, a carpal, a metacarpal, a clavicle, a scapula, a pelvis, a knee joint, an elbow joint, a shoulder joint, a hip joint, an ankle, a vertebra, a spine, etc.

Embodiments of the inventions described herein relate to the design, manufacture, and use of a set of two guides for performing a surgical procedure on a patient. Certain embodiments more specifically relate to the design, manufacture, and use of a set of two guides for performing an osteotomy on a patient. In some embodiments, the two-part surgical guides described herein may be manufactured using additive manufacturing techniques to make the guides patient-specific.

As used herein, the term "patient-specific" relates to any surgical, therapeutic or diagnostic device or tool such as an implant, a prosthesis or a surgical guide which is designed based on an individual patient's anatomy to include features which have a custom fit and/or to perform a customized function for a specific patient. The use of guides and implants which are patient-specific makes it possible to ensure an improved or optimized accuracy of the surgical intervention and an improved anatomical fit for prosthetic structures thereby ensuring optimized functionality for each patient. Even when such devices are used in combination with standard implants, tools, devices, surgical procedures, or other methods, important benefits in accuracy of placement can be obtained.

These two-part surgical guides overcome several of the drawbacks present in current patient-specific guide technologies by decoupling the registration (e.g. fitting) surface of the patient-specific guide from most of the functional elements (e.g. drilling guides, cutting guides, etc.) provided to assist the surgeon. By decoupling the registration surface from the functional elements, the size of each of the individual guide parts can be kept small, which may reduce the size of incision needed for performing a surgical procedure, thereby reducing complications such as healing time and scarring from the surgical procedure..

In some embodiments, the registration surface may refer to one or more contact surfaces on the surgical guide that correspond to and conform to portions (e.g. surfaces) on the patient's anatomy (e.g. bone), such that when the surgical guide is positioned on the patient's anatomy with the registration surfaces contacting the surfaces of the patient's anatomy, the surgical guide is restricted from movement with respect to the patient's anatomy and is accurately positioned on the patient's anatomy. For example, in some embodiments, the registration surface may be designed so as to substantially conform to only a single position on the patient's anatomy.

Not all surfaces on the patient's anatomy may provide the necessary fit and stability when interacting with the surgical guide to ensure the proper positioning of the surgical guide. For example, certain portions of the patient's anatomy may include relatively smooth cylindrical surfaces such that merely having the surgical guide conform to and contact such a smooth surface may not ensure proper positioning of the surgical guide as the surgical guide may easily move. Accordingly, the registration surface may need to be configured to contact the patient's anatomy at multiple surfaces, such as surfaces with more specific features that allow the surgical guide to substantially conform to only a single position on the patient's anatomy. For example, in a high tibia osteotomy ("HTO") procedure, suitable surfaces on the patient's anatomy may include one or more of an anterior surface around the tibial tuberosity, a surface proximal from the attachment of the patellar tendon, and a surface distal from the attachment of the patellar tendon. These multiple surfaces on the patient's anatomy may be on opposite sides of a bone, or at a distance from one another, such that it may significantly increase the bulk of a single-piece patient-specific guide. In particular, a single piece patient-specific guide, as discussed, may then need to both include the functional elements that add bulk, and the registration surfaces that add bulk. Due to this bulky design, it may be difficult to actually position the surgical guide on the patient's anatomy, since the bulky design makes it difficult to maneuver the surgical guide on the patient's anatomy due to other soft tissues at the surgical site. Therefore, the single-piece patient-specific guides may require larger cuts in the soft tissue at the surgical site to position the surgical guide as compared to the embodiments presented herein.

For example, in some embodiments, for a two-part surgical guide, a profile of the guide part with the registration surface may be designed to have a low profile (e.g. reduced dimensions, such as thickness) so the guide part can be placed on the patient's anatomy with minimal disruption to soft tissue near the surgical site. For example, in some embodiments, the guide part (also referred to herein as the first part) may have a profile between 1 to 3 mm. In particular, the low profile of the guide part may allow portions of the guide part to be positioned between the soft tissue and the bone of the patient that the guide part attaches to, therefore avoiding the need to damage or cut the soft tissue. In some embodiments, the guide part may further have tapered (e.g. rounded or chamfered) edges to facilitate the positioning of the guide part between the soft tissue and the bone. The low profile of the guide part may further help facilitate with removal of the guide part from the patient's anatomy by providing flexibility to the guide part to assist in removal. In contrast, current bulky patient-specific guides are too thick to be flexible, and therefore may require additional features such as additional apertures or additional surgical steps such as removal and reinsertion of pins to ensure the guide part can be removed from the patient's anatomy. Such additional features may add cost or reduce effectiveness of the guide as the additional apertures may allow for more movement of surgical tools during surgical procedures. Such additional surgical steps increase surgery time and introduce potential sources of error.

In certain embodiments, a first part of the two-part guide may be designed specifically with fitting stability as its main purpose. As such, the first part of the two-part guide may be designed without many of the functional elements that are typically present on surgical guides, such as osteotomy guides. Further, the first part includes a registration surface to stably fit onto the patient's anatomy. In some embodiments, the first part of the two-part guide may include one, two or more guiding elements (e.g. drill guides) for receiving reference pins (e.g. guide wires). For example, the two-part guide may include two or more drill guides (e.g. drill cylinders) for drilling holes into an anatomy of the patient (e.g. bone) and inserting guide wires, such as k-wires, inside of the holes and into the patient's anatomy. In some embodiments, reference pins may be directly drilled into the bone through the drill guides. In some embodiments, the guide wires are left in the bone when the first part of the two-part guide is removed. As discussed, in some embodiments, the first part of the two-part guide has a flexible design due to it having a low profile/reduced thickness at least in certain portions, to facilitate removal of the first part even with the guide wires inserted in the bone and interacting with the drill cylinders. Further, the low profile of the first part may facilitate positioning the first part on the bone, with reduced damage to soft tissues surrounding the bone.

In some embodiments, the guide wires may be used in performing an osteotomy. In particular, the guide wires may be used for sliding the sawblade against the guide wires to guide the sawblade when performing the osteotomy cut.

A second part of the two-part guide is also provided. The second part of the two-part guide is configured to reference its position on the patient's anatomy based on the guide wires inserted in the patient's anatomy using the first part. By referencing its position based on the inserted guide wires, the need for a large fitting surface is eliminated. In particular, the second part may include apertures (e.g. holes, cylindrical openings, etc.) configured to receive the guide wires and therefore position the second part with respect to the patient's anatomy in a particular position. In particular, in certain aspects the apertures may be sized and shaped to correspond to the guide wires to restrict movement of the second part when the guide wires are inserted. The second part may further include one or more contact surfaces that conform to one or more portions of the underlying anatomy of the patient when positioned on the guide wires and the anatomy of the patient. These contact surfaces may provide additional stability (e.g. prevent wobble) of the second part, but are not sufficient alone (e.g. without the guide wires) to securely position the second part on the patient's anatomy.

Accordingly, the bulk and size required for the second part is reduced, and the second part may only need to conform to a smaller area on the patient's anatomy, thereby reducing the incision required for the surgical site. Further, even if the second part includes some additional bulk (e.g. thickness) to accommodate the functional features (e.g. cut guiding surfaces, drill guides, reamer guides, etc.) used for guiding a procedure (e.g. drilling, cutting, reaming, etc.) on the patient's anatomy as compared to the first portion, the second part does not need to conform to all the surfaces on the patient's anatomy that the first part does. Therefore the extra bulk of the second part may be limited to a smaller area corresponding to a smaller incision at the surgical site. For example, the second part may be designed to not extend beyond the apertures and functional elements of the second part in a direction substantially parallel to a surface of the patient's anatomy by more than, for example, 3mm, 2mm, or 1mm, since the additional extension is not needed for stability, thereby reducing bulk and a footprint of the second part.

Moreover, in some embodiments, the second part of the two-part guide does not include a cut guiding surface (e.g. a cut slot, a sawblade-guiding surface, etc.) for guiding cuts into the bone, as the guide wires left in place after the removal of the first part serve that purpose. Accordingly, the bulk of the second part can be further reduced. In some embodiments, the second part of the two-part guide may still include at least one cut-guiding surface, however, to assist in the surgical procedure, while still providing reduced bulk as compared to current patient-specific surgical guides. For example, if the two-part guide is designed for a closing wedge osteotomy where a portion (e.g. wedge) of the bone is removed, two cuts in the bone may be used, and therefore the second part may include one or more cut-guiding surfaces to guide one or more of the multiple cuts.

In some embodiments, instead of adding bulk and including multiple cut-guiding surfaces for procedures utilizing multiple cuts, the first part of the two-part guide may include multiple drilling guides positioned on the first part such that when the first part is positioned on the bone, the drilling guides align with positions where the multiple cuts are performed. For example, two or more drilling guides may be aligned at each cut position. The drilling guides may be used to guide drilling into the bone and insertion of guide wires into the bone. The first part may then be removed, and the cuts performed using the guide wires to guide the cuts as discussed herein.

As described, the second part may include one or more functional elements of a suitable type depending on the procedure to be performed. In some embodiments, the functional elements may comprise a radiopaque material that can be seen on a fluoroscopy (real-time use of an imaging technique such as X-rays) and used to check on the status of the surgery in real time.

Certain embodiments described herein are described with respect to osteotomies and accordingly, two-part guides for osteotomies. Accordingly, certain described aspects may pertain specifically to osteotomies and provide certain benefits over current guides and techniques for performing osteotomies, such as the configuration of functional elements on the guides specifically for performing osteotomies. However, certain described aspects may also be used for two-part guides for other surgical procedures, as would be apparent to one of skill in the art. Accordingly, certain embodiments herein may relate to two-part guides for surgical procedures other than osteotomies as well as osteotomies.

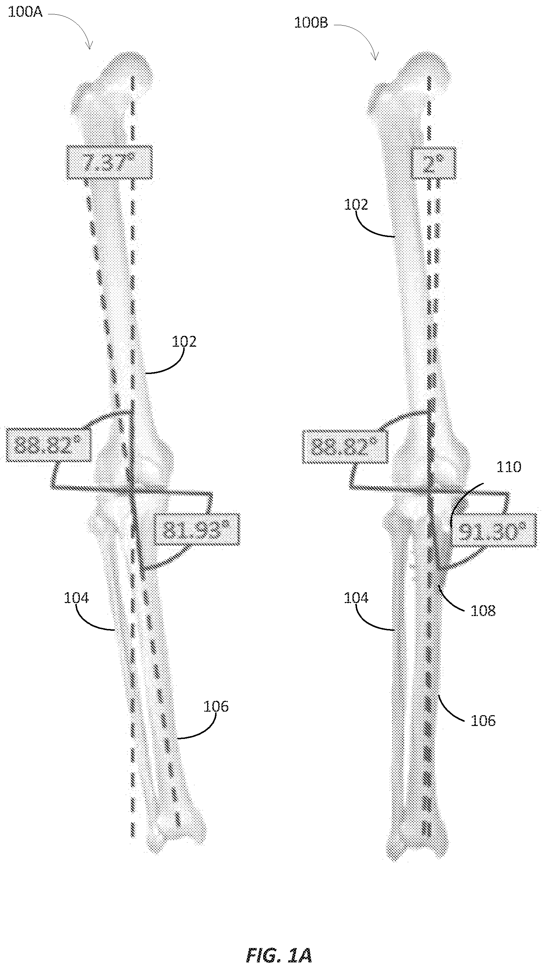

Figure 1A illustrates 3-D models of a bone of a patient that is to undergo surgery, in accordance with certain embodiments. In this particular example, the surgical procedure to be performed is an osteotomy for a proximal tibia. In particular, Figure 1A illustrates a pre-surgery bone anatomy 100A and a desired bone anatomy 100B that the surgery is trying to achieve. The 3-D models may be used as part of a preplanning of the surgical procedure using virtual 3-D technology. In this particular example, the 3-D reconstruction plan including virtual 3-D models of the pre-surgery bone anatomy 100A and the desired bone anatomy 100B is generated by a computing device based on CT data acquired using a suitable scan program and a suitable CT scanner. However, as discussed herein, a 3-D model may be generated using other appropriate imaging techniques (e.g. MRI, X-rays, etc.)

As shown, the 3-D models include images of various bones in the leg of the patient. The pre-surgery bone anatomy 100A includes the femur 102. At the distal end of the femur 102 is the knee joint, which joins the femur to the fibula 104 and the tibia 106. The mechanical axes and angles of the pre-surgery bone anatomy 100A are computed and may be displayed on a computer monitor for evaluation by a surgeon and/or a clinical engineer.

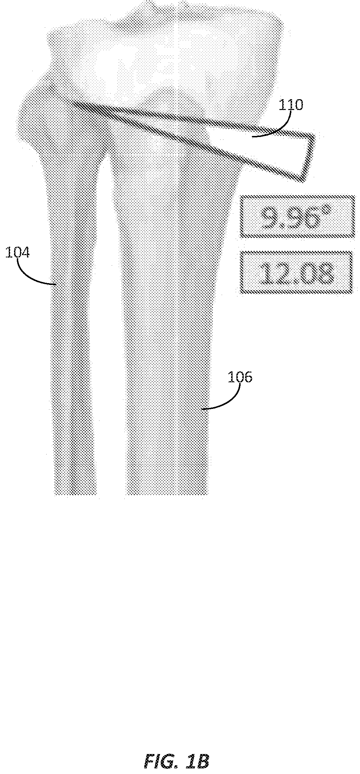

The desired bone anatomy 100B shows how the angle of the tibia with respect to the femur will change after completion of the osteotomy procedure. Using the scanned CT data, a desired angle of correction may be determined for the bone anatomy, and the mechanical axes upon which the bone will be cut and realigned may be defined as shown. In this example, the desired bone anatomy 100B is achieved by removing a wedge 110 out of the proximal tibia and supporting the modified bone using an osteosynthesis plate 108. Figure 1B illustrates a close-in view of the planned osteotomy cut shown in Figure 1A, in accordance with certain embodiments. As shown, a hinge axis and wedge 110 have been planned which will realign the tibia 106 based on the size and location of the cut made through the bone.

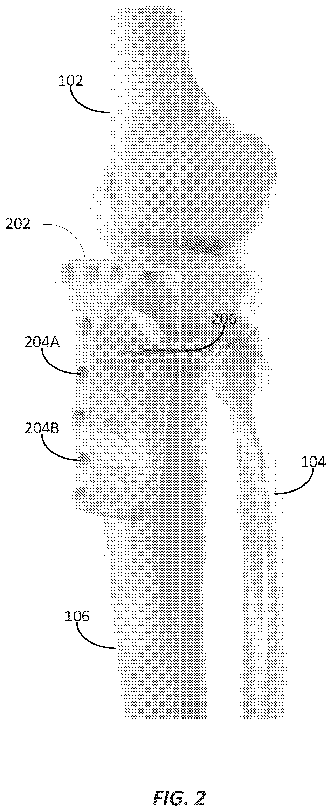

Based on the surgical plan developed based on the measurements and analysis performed in connection with Figures 1A and 1B, a patient-specific surgical guide may be designed and manufactured to assist the surgeon in drilling holes and performing the osteotomy cut in the proper location. Figure 2 provides an illustration of a bulky single-piece patient-specific osteotomy guide 202, which suffers from some of the drawbacks discussed herein. In this example, the patient-specific surgical guide 202 is used in a high tibia osteotomy ("HTO") procedure, and is shown as placed over the bone of the patient. The patient-specific surgical guide 202 may include two types of functional features. The first type of functional feature is one or more drill cylinders such as, for example, drill cylinder 204A and drill cylinder 204B. The drill cylinders may be used for pre-drilling the holes for fixation screws of an osteosynthesis plate. During the procedure, the drill cylinders guide a drill bit into the bone of the patient so that holes for receiving fixation screws through the osteosynthesis plate are located properly, and the osteosynthesis plate is placed in the optimal position on the bone of the patient.

The patient-specific surgical guide 202 may also include one or more cut slots such as cut slot 206. The cut slot 206 may be designed to guide a sawblade into the bone so that the osteotomy cut is both in the proper location and made to the proper depth inside of the bone. Using this guide, the surgeon can pre-drill screw holes before making the osteotomy cut. The inventors have recognized that while the guide shown in Figure 2 provides a good and stable fit on the bone, in order to achieve that fit and stability, they need a relatively large footprint. This is because the guides need to fit on roughly cylindrical bones, and the guides need to make contact with a large surface area in order to find sufficient bony features that can offer the needed stability. The footprint becomes even larger because it needs to accommodate all the functional features as discussed herein.

The large footprint of the conventional single-piece patient-specific guides such as guide 202 often require that the guide be placed or fitted on regions where surgeons are reluctant to clear soft tissue. In addition, the inventors have discovered that the location of the cut slots, derived from the surgical planning process can often end up being too far in posterior. Existing guides such as guide 202 also provide little visibility on the fitting surface and require, due to their size, a larger incision than is preferred. These factors can result in difficulty in positioning the guide correctly. If the guide is not positioned correctly, deviations from the planned correction may result in the course of carrying out the surgical procedure. The inventors have further recognized that the use of cut slots to guide the osteotomy cut may impose a requirement that a thickness of the sawblade be known during the design of a guide, and also may result in a surgical technique that is dissimilar from traditional osteotomy techniques. Finally, the surgical guide shown in Figure 2 does not include any features that allow double checking the status of various parameters (e.g. cut positions, bone positions, drill positions, alignments, etc.) during surgery using fluoroscopy, if desired.

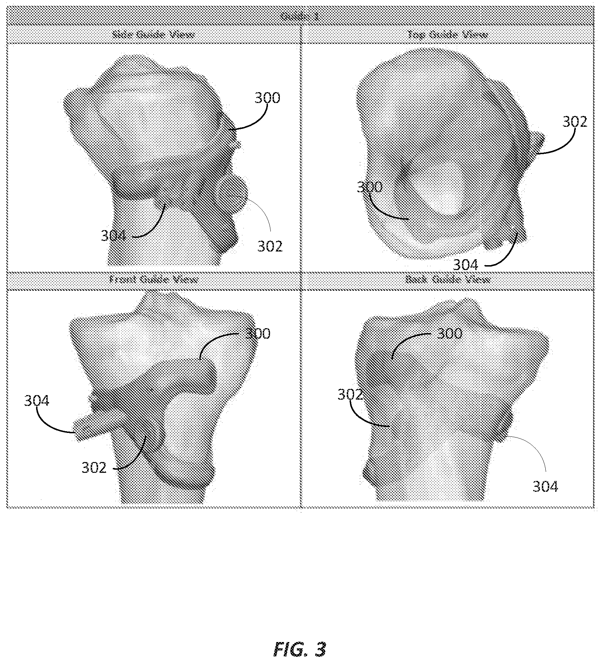

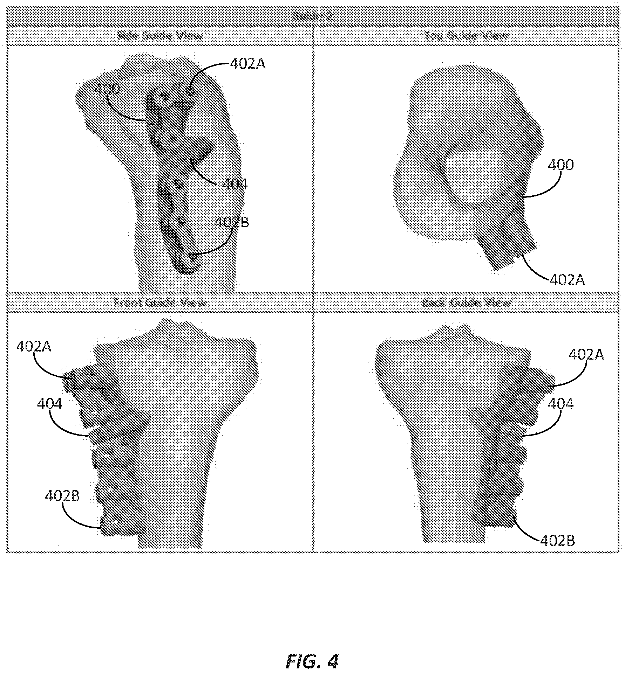

Having recognized the various problems with existing patient-specific guide designed, the inventors have devised an approach in which two guide parts are used separately, as described herein. Figures 3 and 4 provide illustrations of a first part and a second part of a two-part guide which may be used in accordance with one or more embodiments of the invention. As is explained herein, the first guide provides the stability needed to allow the user to position reference pins in the patient's anatomy (e.g. bone), for example along a planned osteotomy plane, while the second guide slides over the reference pins (e.g. guide wires) (using the reference pins for stability) and includes at least one functional element to allow a user to perform a guided procedure on the anatomy of the patient, such as pre-drill the holes for fixation elements (e.g. screws) of an osteosynthesis implant (e.g. an osteosynthesis plate). Further, the reference pins can be used by the surgeon for performing the osteotomy, for example by sliding a sawblade against the reference pins.

Figure 3 provides a front, a side, a top, and a back side view of a first guide 300 in a two-part surgical guide. In some embodiments, the first guide 300 is an osteotomy guide used for performing an osteotomy. As noted above, both parts of the two-part guide may be manufactured using additive manufacturing technologies such as selective laser sintering or stereolithography. As compared to the guide 202 shown in Figure 2, the first guide 300 has a significantly smaller footprint in the surgical site. In some embodiments, the first part 300 of the two-part guide includes a push feature 302. The push feature 302 may be used to place the first guide part 300 in the appropriate position on the anatomy of the patient, such as the tibia 106 of a patient. The push feature may be a pressure point feature such as those described in co-owned U.S. Patent No. 8,984,731. Unlike the guide 202 from Figure 2 which has several drill cylinders configured for drilling holes to receive fixation elements to fix an osteosynthesis implant to the bone (e.g. or perform other surgical procedures), the first part 300 of the two-part guide shown in Figure 3 includes only drill cylinders 304 (e.g. only two drill cylinders) that are used to position guide wires into the bone and not other functional features such as to make cuts or drill holes, such as for fixation elements to fix an osteosynthesis implant to the bone. These drill cylinders 304 are used to drill holes which receive guide wires (such as k-wires, for example), or are used to receive reference pins that directly drill into the patient's anatomy after the first guide part 300 has been placed in the proper location on the bone.

Although not specifically called out in Figure 3, the bottom surface of the guide part 300 has been designed and manufactured to conform to the surface of the patient's anatomy, such as the tibial anatomy as shown, to ensure a snug and stable fit. However, because this guide part is primarily used only to drill holes for the guide wire through the drill cylinders 304, its footprint can be relatively small. The smaller footprint makes it easier to place the part 300 properly. In some embodiments, use of metal drill sleeves can be abandoned to reduce the size of the drill cylinders even further. Once the guide wire holes have been drilled, the guide wires may be placed into the bone, and the guide part 300 may be slid off of the wires and removed from the surgical site. In some embodiments, guide wires with diamond or trocar tips may be used and drilled directly into the bone. In such embodiments, pre-drilling guide wire holes may not be made in the patient's anatomy.

The second part of the two-part osteotomy guide may be inserted into the patient (e.g. once the first guide part 300 is removed). Figure 4 provides a front, a side, a top, and a back side view of an exemplary second guide part 400 in a two-part surgical guide according to certain embodiments. Unlike the first guide part 300 which is primarily used for its stability and promotes the necessary precision and accuracy in the location of the guide wires, the second guide part 400 relies more on the guide wires for location and stability. Because the second guide part 400 does not need to rely upon patient anatomy to provide as much stability, its footprint can be reduced. The second guide part 400, supported by the inserted guide wires, provides the necessary drill cylinders (or other functional features) to assist in guiding a procedure on the anatomy of the patient, such as the fixation of an osteosynthesis plate.

In the particular example shown in Figure 4, the second guide part 400 includes several drill cylinders such as drill cylinders 402A and 402B. These drill cylinders are positioned such that the holes drilled through them will be appropriately spaced to receive fixation elements (e.g. screws) for an osteosynthesis implant (e.g. osteosynthesis plate). The second guide part 400 may be placed onto the patient's anatomy (e.g. tibial anatomy) by sliding the inserted guide wires through the apertures 404 located in the central portion of the guide part 400. As discussed above, this may be the approximate location where the osteotomy cut is to be made. As with the guide part 300, the underneath surface of the second guide part 400 may be manufactured to conform to the anatomical surface of the patient, such as the tibia, to provide further stability when slid over the guide wires.

In some embodiments, such as for a second guide part for a closing wedge osteotomy, the second guide part may include a cut-guiding surface, such as a cut slot. An osteotomy may be performed using the cut-guiding surface for guidance, e.g. by sliding a sawblade along the cut-guiding surface.

In some embodiments, an osteotomy cut may be made using the guide wires to guide the sawblade, e.g. after the second guide part has been removed. Using guide wires instead of a cut slot (as shown in Figure 2) allows the surgeon to use a more familiar technique.

As discussed herein, the first part of a two-part surgical guide may include one or more contact surfaces that allow for placement of the first part on an anatomical part in a particular position for a snug and secure fit. Such contact surfaces can be determined which allow the first part to fit specifically onto the anatomical part of the patient. Further, in some embodiments, based on a stability analysis thereof the location and extent (e.g. size, number, etc.) of the contact surfaces and the location of a push feature can be evaluated and determined which will allow to optimize positioning and stability of the first part.

Accordingly, in some embodiments, a three-dimensional model of the patient's anatomy to undergo surgery is generated from medical images of the patient such as X-ray, Magnetic Resonance Imaging (MRI), Positron Emission Tomography (PET) scan, Computed Tomography (CT) scan, ultrasound images, etc. A patient-specific first part design can be determined based on the three-dimensional model.

For example, one or more contact surfaces can be determined based on images of the anatomical part of said patient, the three-dimensional model, and/or pre-operative planning of the surgical procedure. Typically, the contact surfaces of the patient-specific first part are patient-specific, i.e. the contact surfaces typically have a shape which is conformal with at least a part of a specific patient's anatomical part.

From the contact surfaces, the stability of the patient-specific first part when there is contact between the contact surfaces and the patient's anatomical part can be determined. The geometric information of the contact surfaces, including the vertex coordinates and unit outward normal vectors of the faces of the contact surfaces can be determined. This geometric information can then be used to characterize the stiffness of the contact between the patient-specific first part and the anatomy. This stiffness can be understood to be the resistance the contact between the patient-specific first part and the anatomy provides towards an externally applied force. The stiffness information can then serve as input for identifying the least-constrained direction of translation and rotation of the patient-specific first part on the anatomical part. In some embodiments, the stiffness information can also serve to evaluate whether other, greater or more suitable contact surfaces should be determined.

More particularly, in some embodiments, the contact surface(s) between the patient-specific first part and the patient's anatomy is identified. Using the points defining this surface and their corresponding unit outward normal vectors, a spatial stiffness matrix of the contact is calculated. Using the eigenvalues of this stiffness matrix, information about the translational and rotational stiffness of the contact can be retrieved. The eigenvectors corresponding to the smallest eigenvalues will define the least-constrained axes of the contact surface.

In this way, in some embodiments, the least-constrained direction for a translation and/or rotation of the patient-specific first part on the anatomical part is determined thereby identifying the optimal direction that force can be applied to the patient-specific first part upon positioning the patient-specific first part on the anatomical part. In some embodiments, this information is used to determine the position and orientation to provide one or more push feature on the design of the patient-specific first part.

Thus, in certain embodiments, a push feature can be included on the patient-specific first part to restrict a possible straight movement over the least-constrained direction of translation. To do this, the force direction of the push feature may be oriented perpendicular to the least-constrained direction of translation. Similarly, in some embodiments, a push feature can be added to the patient-specific first part to restrict a likely rotation around the instantaneous axis of rotation defined by the least-constrained axis of rotation. The functional element may then be positioned such that the force direction is parallel to the least-constrained axis of rotation. In some embodiments, the position of the functional element is as far as possible from the location of instantaneous axis of rotation, to create a maximal restrictive moment applied on the patient-specific first part.

In certain embodiments, the axis of least-constrained direction for rotation and translation can be determined using finite element analysis.

As used herein, the term "push feature" refers to a feature that is provided on the patient-specific first part according to the present methods wherein the push feature allows the user to apply force onto the patient-specific first part. This allows the user to specifically and correctly position the patient-specific first part onto the pre-defined location of the anatomical part. In particular embodiments, it further allows the operator to maintain the position of a patient-specific first part in the correct pre-defined location without any major additional burden for the operator. In particular embodiments, the push feature can also be designed to enhance the application of a force by the user. Typically, the force is a manual force, more particularly a manual force which is created by the operator pushing on the device. The location and orientation of the push feature can be determined in such a way that application of a force thereto ensures stability of the patient-specific first part in the desired orientation. In particular embodiments, the push feature may be a dedicated push feature. In certain embodiments, the push feature may be a handle. In certain embodiments, the push feature may be designed to receive one or more fingers, more particularly finger tips. In further particular embodiments the push feature is a "finger pit".

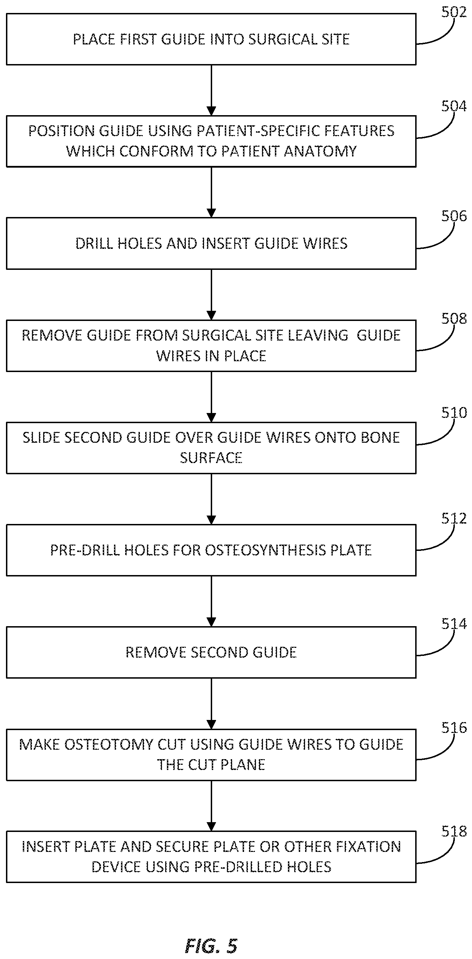

Figure 5 is a flow chart illustrating a high-level process for performing a surgery using a two-part surgical guide such as the guides shown in Figures 3 and 4 above. In particular, Figure 5 illustrates a process for performing an osteotomy surgery. The process begins at block 502, where the first part of the two-part guide is placed within the surgical site. As noted above, the first part of the two-part guide may have patient-specific features (e.g. contact surfaces) which conform to at least a portion of the anatomical surface in the patient, such as to substantially restrict movement of the first part with respect to the anatomical surface when positioned on the anatomical surface. Further, at block 504, the first part of the guide is positioned based on the features which conform to the patient's anatomy. In some embodiments, such as where the first part of the guide has a low profile and optionally tapered, rounded or chamfered edges, the first part of the guide can be positioned onto the patient's anatomy by sliding the first part of the guide between a soft tissue and a bone of the patient.

Continuing at block 506, where holes are drilled and guide wires are inserted into the bone. In some embodiments, instead of drilling holes and inserting guide wires into the bone, as discussed, the guide wires may be directly drilled into the bone. Further, in some embodiments, instead of both drilling holes and inserting guide wires into the bone at block 506, only holes may be drilled into the bone at block 506. The guide wires may then be inserted into the bone after the first guide part is removed from the bone (at block 508) and before sliding the second guide part over the guide wires (at block 510). As noted above, the guide may be designed during the planning process described in Figures 1A and 1B so that the guide wires are inserted on the plane of the planned osteotomy cut. As further discussed above, the holes may be drilled through drill cylinders (e.g. metal drill cylinders) in some embodiments. Other embodiments may not include them.

Once the guide wires have been inserted, the process may then continue to block 508. At block 508, the first guide part may be removed from the surgical site by backing it out over the guide wires. The guide wires stay in place. In some embodiments, the removal of the first guide part is facilitated by the first guide part having at least one flexible portion that allows the first part to flex so that features which conform to the patient's anatomy and constitute an undercut with respect to the orientation of the guide wires are disengaged from the patient's anatomy while the guide wires stay in place. Next, at block 510, the second part of the two-part osteotomy guide is slid along the guide wires onto the surface of the bone. As noted above, the second guide part may have apertures specifically designed to receive the guide wires, and in doing so, positions the remaining drill cylinders or other functional elements on the second guide part correctly with respect to the patient's anatomy. Next, at block 512, holes for the fixation device (such as an osteosynthesis plate or wedge) are pre-drilled through the drill cylinders of the second guide part. Further, in some embodiments, where the second guide part includes a cut-guiding surface to guide an osteotomy cut, an osteotomy cut is performed using the cut-guiding surface to guide a saw blade (or other cutting device) along the cut plane. Once the holes have been drilled, the second guide part is removed at block 514. Once the second guide part has been removed, at block 516, an osteotomy cut is made using the guide wires to guide a saw blade (or other cutting device) along the cut plane. Once the cut has been made, at block 518, the bone is repositioned and the osteosynthesis plate (or other type of fixation device) is inserted into the patient and secured to the bone using fixation elements (e.g. screws) inserted into the pre-drilled holes.

In certain embodiments, the osteosynthesis plate or other osteosynthesis implant secured to the patient includes a plurality of apertures configured to receive the fixation elements and secure the osteosynthesis implant to the bone. In certain aspects, certain apertures, such as a first aperture, are configured to be positioned at a position on a first side (e.g. top or bottom) of the osteotomy cut (e.g. on a first side of an osteotomy plane corresponding to the osteotomy cut). In certain aspects, certain apertures, such as a second aperture, are configured to be positioned at a position on a second side (e.g. the other of the top or bottom) of the osteotomy cut (e.g. on a second side of an osteotomy plane corresponding to the osteotomy cut). These first and second apertures on the osteosynthesis plate correspond to drilling guides in the second guide part, such that the second guide part can be used to drill holes in the bone to receive the fixation elements that interact with the first and second apertures. Therefore, the first aperture and second aperture, when secured to the bone, are aligned with the positions on the bone corresponding to holes drilled through drill guides of the second guide part.

However, the distance between the first aperture and second aperture on the osteosynthesis plate may not be equal to the distance between the corresponding drill guides on the second guide part for drilling the holes the first aperture and second aperture align with. In particular, as discussed, after the holes are drilled in the bone utilizing the second guide part and the osteotomy cut is made, the portions of the bone on either side of the osteotomy cut may be moved with respect to one another to shorten or lengthen or change the alignment of the bone. Accordingly, the distance between the drilled holes changes as the portions of the bone are moved with respect to one another. The distance between/angle of the first and second aperture in the osteosynthesis implant may therefore be designed to align with the holes drilled in the bone when the portions of the bone are in the desired changed position as opposed to the original position before the surgery. The corresponding drill guides in the second guide part, however, may have a distance between/angle that aligns with the holes drilled in the bone when the portions of the bone are in the original position before the surgery.

Using the two-part guide design described above provides a number of advantages not available in current guides and techniques. By providing less bulky guides, a smaller fitting surface is defined, and a smaller incision may be used (promoting a faster, less painful recovery). In addition, the two-part guide allows for high visibility because its footprint does not block a large portion of the surgical site. Additionally, by using the guide wires to define the cut plane, the surgical technique is closer to osteotomy procedures to which surgeons are accustomed. This results in a shorter learning curve for surgeons. Finally, by using the guide wires, it becomes easier to check status under fluoroscopy, because the guide wires show up on fluoroscopy and are a clear indication of the cutting plane.

The patient-specific two-part surgical guides described herein may be manufacturing utilizing various additive manufacturing and/or three-dimensional (3D) printing systems and techniques. Typically, additive manufacturing techniques start from a digital representation of the 3D object to be formed. Generally, the digital representation is divided into a series of cross-sectional layers, or "slices," which are overlaid to form the object as a whole. The layers represent the 3D object, and may be generated using additive manufacturing modeling software executed by a computing device. For example, the software may include computer-aided design and manufacturing (CAD/CAM) software. Information about the cross-sectional layers of the 3D object may be stored as cross-sectional data. An additive manufacturing (e.g. 3D printing) machine or system utilizes the cross-sectional data for the purpose of building the 3D object on a layer by layer basis. Accordingly, additive manufacturing allows for fabrication of 3D objects directly from computer generated data of the objects, such as computer aided design (CAD) files or STL files. Additive manufacturing provides the ability to quickly manufacture both simple and complex parts without tooling and without the need for assembly of different parts.

Additive manufacturing processes generally include providing energy from an energy source (e.g. a laser, an electron beam, etc.) to solidify (e.g. polymerize) layers of building material (e.g. plastic, metal, etc.). For example, the additive manufacturing machine may selectively apply energy from an energy source to (e.g. scan) the building material based on a job file. The job file may include information regarding slices of a digital representation of an object to be built using an additive manufacturing process.

An additive manufacturing machine builds an object on a layer-by-layer basis by applying energy to (e.g. scanning) the layers of building material according to the scanning pattern for each individual layer as indicated in a job file. For example, the additive manufacturing machine may scan a first layer of physical building material corresponding to a first slice of a digital representation of an object according to the scanning pattern for the first slice. The additive manufacturing machine may then scan a second layer of building material corresponding to a second slice adjacent to the first slice according to the scanning pattern for the second slice. The additive manufacturing machine continues scanning layers of building material corresponding to all the slices in the job file, until the layer corresponding to the last slice is scanned.

Selective laser sintering (LS) is an additive manufacturing technique used for 3D printing objects. LS apparatuses often use a high-powered laser (e.g. a carbon dioxide laser) to "sinter" (i.e. fuse) small particles of plastic, metal, ceramic, glass powders, or other appropriate materials into a 3D object. The LS apparatus may use a laser to scan cross-sections on the surface of a powder bed in accordance with a CAD design or job file. Also, the LS apparatus may lower a manufacturing platform by one layer thickness after a layer has been completed and add a new layer of material in order that a new layer can be formed. In some embodiments, an LS apparatus may preheat the powder in order to make it easier for the laser to raise the temperature during the sintering process.

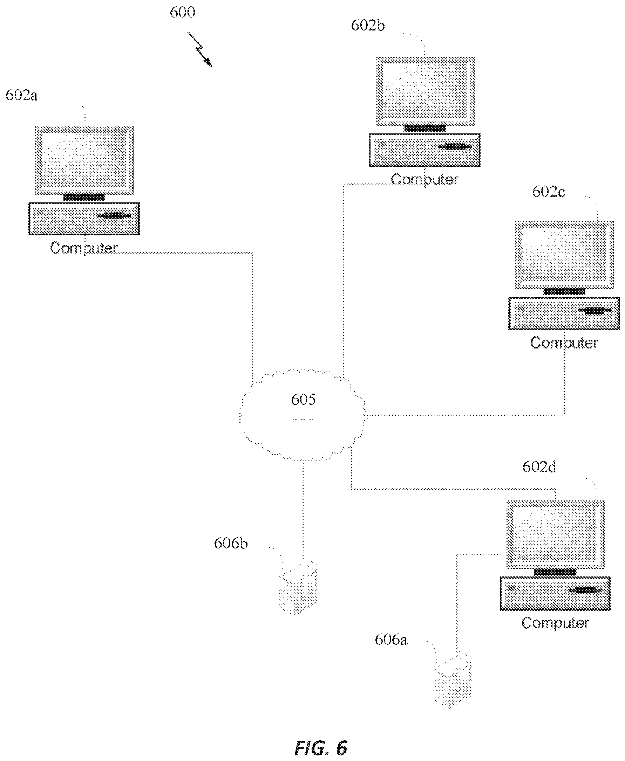

Embodiments of the invention, including two-part surgical guides, may be designed and manufactured within a system for designing and manufacturing 3D objects. Turning to Figure 6, an example of a computer environment suitable for the implementation of 3D object design and manufacturing is shown. The environment includes a system 600. The system 600 includes one or more computers 602a-602d, which can be, for example, any workstation, server, or other computing device capable of processing information. In some aspects, each of the computers 602a-602d can be connected, by any suitable communications technology (e.g. an internet protocol), to a network 605 (e.g. the Internet). Accordingly, the computers 602a-602d may transmit and receive information (e.g. software, digital representations of 3-D objects, commands or instructions to operate an additive manufacturing device, etc.) between each other via the network 605.

The system 600 further includes one or more additive manufacturing devices (e.g. 3-D printers) 606a-606b. As shown the additive manufacturing device 606a is directly connected to a computer 602d (and through computer 602d connected to computers 602a-602c via the network 605) and additive manufacturing device 606b is connected to the computers 602a-602d via the network 605. Accordingly, one of skill in the art will understand that an additive manufacturing device 606 may be directly connected to a computer 602, connected to a computer 602 via a network 605, and/or connected to a computer 602 via another computer 602 and the network 605.

It should be noted that though the system 600 is described with respect to a network and one or more computers, the techniques described herein also apply to a single computer 602, which may be directly connected to an additive manufacturing device 606. Any of the computers 602a-602d may be configured to design and/or manufacture two-part surgical guides as described herein.

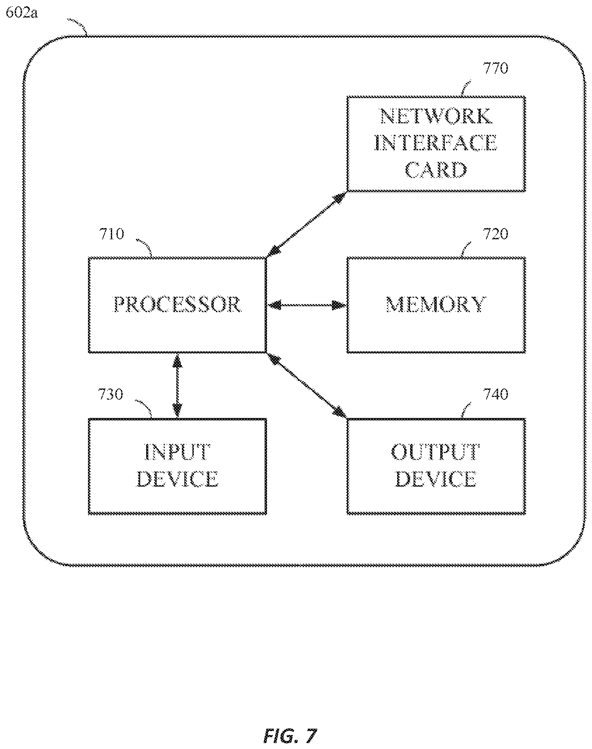

Figure 7 illustrates a functional block diagram of one example of a computer of Figure 6. The computer 602a includes a processor 710 in data communication with a memory 720, an input device 730, and an output device 740. In some embodiments, the processor is further in data communication with an optional network interface card 770. Although described separately, it is to be appreciated that functional blocks described with respect to the computer 602a need not be separate structural elements. For example, the processor 710 and memory 720 may be embodied in a single chip.

The processor 710 can be a general purpose processor, a digital signal processor (DSP), an application specific integrated circuit (ASIC), a field programmable gate array (FPGA) or other programmable logic device, discrete gate or transistor logic, discrete hardware components, or any suitable combination thereof designed to perform the functions described herein. A processor may also be implemented as a combination of computing devices, e.g. a combination of a DSP and a microprocessor, a plurality of microprocessors, one or more microprocessors in conjunction with a DSP core, or any other such configuration.

The processor 710 can be coupled, via one or more buses, to read information from or write information to memory 720. The processor may additionally, or in the alternative, contain memory, such as processor registers. The memory 720 can include processor cache, including a multi-level hierarchical cache in which different levels have different capacities and access speeds. The memory 720 can also include random access memory (RAM), other volatile storage devices, or non-volatile storage devices. The storage can include hard drives, optical discs, such as compact discs (CDs) or digital video discs (DVDs), flash memory, floppy discs, magnetic tape, and Zip drives.

The processor 710 also may be coupled to an input device 730 and an output device 740 for, respectively, receiving input from and providing output to a user of the computer 602a. Suitable input devices include, but are not limited to, a keyboard, buttons, keys, switches, a pointing device, a mouse, a joystick, a remote control, an infrared detector, a bar code reader, a scanner, a video camera (possibly coupled with video processing software to, e.g. detect hand gestures or facial gestures), a motion detector, or a microphone (possibly coupled to audio processing software to, e.g. detect voice commands). Suitable output devices include, but are not limited to, visual output devices, including displays and printers, audio output devices, including speakers, headphones, earphones, and alarms, additive manufacturing devices, and haptic output devices.

The processor 710 further may be coupled to a network interface card 770. The network interface card 770 prepares data generated by the processor 710 for transmission via a network according to one or more data transmission protocols. The network interface card 770 also decodes data received via a network according to one or more data transmission protocols. The network interface card 770 can include a transmitter, receiver, or both. In other embodiments, the transmitter and receiver can be two separate components. The network interface card 770, can be embodied as a general purpose processor, a digital signal processor (DSP), an application specific integrated circuit (ASIC), a field programmable gate array (FPGA) or other programmable logic device, discrete gate or transistor logic, discrete hardware components, or any suitable combination thereof designed to perform certain functions described herein.

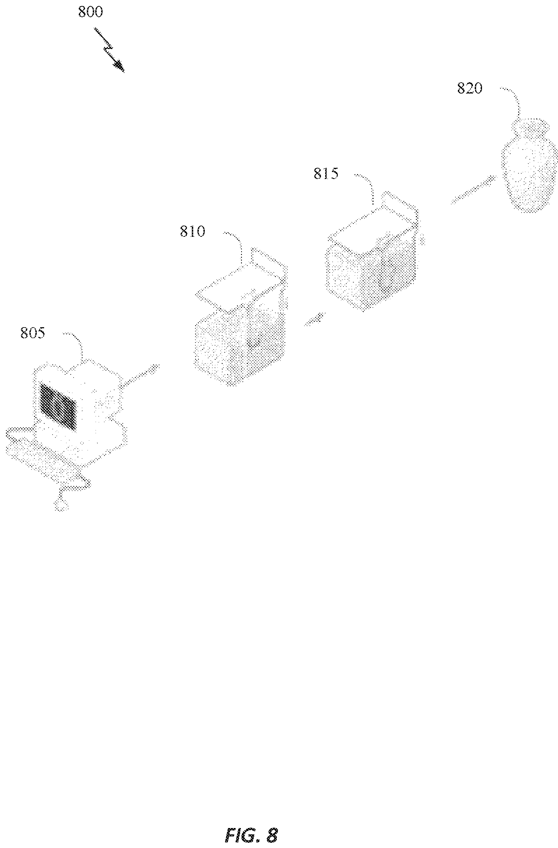

Figure 8 illustrates a process 800 for manufacturing a 3-D object or device. As shown, at a step 805, a digital representation of the object is designed using a computer, such as the computer 602a. For example, 2-D or 3-D data may be input to the computer 602a for aiding in designing the digital representation of the 3-D object. Continuing at a step 810, information is sent from the computer 602a to an additive manufacturing device, such as additive manufacturing device 606, and the device 606 commences the manufacturing process in accordance with the received information. At a step 815, the additive manufacturing device 606 continues manufacturing the 3-D object using suitable materials, such as a liquid resin. At a step 820, the object is finally built.

These suitable materials may include, but are not limited to a photopolymer resin, polyurethane, methyl methacrylate-acrylonitrile-butadiene-styrene copolymer, resorbable materials such as polymer-ceramic composites, etc. Examples of commercially available materials are: DSM Somos^{®} series of materials 7100, 8100, 9100, 9420, 10100, 11100, 12110, 14120 and 15100 from DSM Somos; ABSplus-P430, ABSi, ABS-ESD7, ABS-M30, ABS-M30i, PC-ABS, PC ISO, PC, ULTEM 9085, PPSF and PPSU materials from Stratasys; Accura Plastic, DuraForm, CastForm, Laserform and VisiJet line of materials from 3-Systems; the PA line of materials, PrimeCast and PrimePart materials and Alumide and CarbonMide from EOS GmbH. The VisiJet line of materials from 3-Systems may include Visijet Flex, Visijet Tough, Visijet Clear, Visijet HiTemp, Visijet e-stone, Visijet Black, Visijet Jewel, Visijet FTI, etc. Examples of other materials may include Objet materials, such as Objet Fullcure, Objet Veroclear, Objet Digital Materials, Objet Duruswhite, Objet Tangoblack, Objet Tangoplus, Objet Tangoblackplus, etc. Another example of materials may include materials from the Renshape 5000 and 7800 series.

Various embodiments disclosed herein provide for the use of a controller or computer control system. A skilled artisan will readily appreciate that these embodiments may be implemented using numerous different types of computing devices, including both general-purpose and/or special-purpose computing-system environments or configurations. Examples of well-known computing systems, environments, and/or configurations that may be suitable for use in connection with the embodiments set forth above may include, but are not limited to, personal computers, server computers, handheld or laptop devices, multiprocessor systems, microprocessor-based systems, programmable consumer electronics, network PCs, minicomputers, mainframe computers, distributed computing environments that include any of the above systems or devices, and the like. These devices may include stored instructions, which, when executed by a microprocessor in the computing device, cause the computer device to perform specified actions to carry out the instructions. As used herein, instructions refer to computer-implemented steps for processing information in the system. Instructions can be implemented in software, firmware or hardware and include any type of programmed step undertaken by components of the system.

A microprocessor may be any conventional general-purpose single- or multi-chip microprocessor such as a Pentium^{®} processor, a Pentium^{®} Pro processor, a 8051 processor, a MIPS^{®} processor, a Power PC^{®} processor, or an Alpha^{®} processor. In addition, the microprocessor may be any conventional special-purpose microprocessor such as a digital signal processor or a graphics processor. The microprocessor typically has conventional address lines, conventional data lines, and one or more conventional control lines.

Aspects and embodiments of the inventions disclosed herein may be implemented as a method, apparatus or article of manufacture using standard programming or engineering techniques to produce software, firmware, hardware, or any combination thereof. The term "article of manufacture" as used herein refers to code or logic implemented in hardware or non-transitory computer readable media such as optical storage devices, and volatile or non-volatile memory devices or transitory computer readable media such as signals, carrier waves, etc. Such hardware may include, but is not limited to, field programmable gate arrays (FPGAs), application-specific integrated circuits (ASICs), complex programmable logic devices (CPLDs), programmable logic arrays (PLAs), microprocessors, or other similar processing devices.

## Claims

1. A method of designing a patient-specific system for performing an osteotomy on a bone of a patient, the patient-specific system comprising:
i) a first guide (300) comprising:
at least one contact surface configured to conform to at least one portion of the bone such that when the at least one contact surface is positioned on the at least one portion of the bone, movement of the first guide (300) is substantially restricted with respect to the bone; and
a plurality of guiding elements (304) aligning with a cut plane and configured to guide placement of a plurality of reference pins in the bone and on the cut plane; and
ii) a second guide (400), separate from the first guide, the second guide comprising:
a plurality of apertures ( 404) each with a shape corresponding to a reference pin, each of the plurality of apertures (404) configured to receive one of the plurality of reference pins upon sliding the second guide ( 400) over the reference pins after removal of the first guide (300) and substantially restrict movement of the second guide (400) with respect to the bone when the plurality of reference pins are received; and
at least one functional element (402A, 402B) for drilling a hole in the bone,
wherein the method comprises a computer-implemented step of:
determining the cut plane in accordance with a plan to, during the osteotomy, cut the bone of the patient by sliding a saw blade against the plurality of reference pins.

2. The method of claim 1, wherein the plurality of reference pins comprise a plurality of guide wires configured to guide the saw blade for performing the osteotomy.

3. The method of claim 1, wherein the plurality of guiding elements (304) comprises a drill guide.

4. The method of claim 1, wherein the second guide (400) further comprises a first contact surface configured to conform to at least a second portion of the bone when the plurality of reference pins are received, wherein the first contact surface is configured such that contact between the first contact surface and the bone alone is insufficient to substantially restrict movement of the second guide ( 400) with respect to the bone.

5. The method of claim 1, wherein the at least one functional element (402A, 402B) comprises a drill guide.

6. The method of claim 1, further comprising an osteosynthesis implant, wherein the osteosynthesis implant optionally comprises a plate.

7. The method of claim 6, wherein the osteosynthesis implant comprises a first aperture and a second aperture, wherein each of the first aperture and the second aperture is configured to receive an implant fixation element, wherein the at least one functional element comprises a plurality of drill guides, and wherein the first aperture and the second aperture are each configured to align with a position on the bone corresponding to a position on the bone of one of the plurality of drill guides when the plurality of reference pins are received by the plurality of apertures.

8. The method of claim 7, wherein:
the first aperture and the second aperture are separated by a first distance, and wherein a first drill guide corresponding to the first aperture and a second drill guide corresponding to the second aperture are separated by a second distance, wherein the first distance is different than the second distance, or
wherein the first aperture is associated with a first bone position that is on a first side of the cut plane and wherein the second aperture is associated with a second bone position that is on a second side of the cut plane, the first side being opposite the second side.

9. The method of claim 1, wherein the at least one functional element comprises a cut-guiding surface and/or one or more radiopaque elements.

10. The method of claim 1, wherein the first guide (300) comprises tapered edges and/or the first guide (300) conforms to a profile between 1 to 3 mm.

11. The method of claim 1, wherein the second guide (400) does not extend beyond the plurality of apertures (404) and the at least one functional element by more than 3 mm.

12. The method of claim 1, wherein the first guide (300) comprises flexible portions for removing the first guide (300) from the bone with the reference pins placed in the plurality of guiding elements (304).

13. A method of manufacturing a patient-specific system for performing an osteotomy on a bone of a patient, the patient-specific system comprising:
i) a first guide (300) comprising:
at least one contact surface configured to conform to at least one portion of the bone such that when the at least one contact surface is positioned on the at least one portion of the bone, movement of the first guide (300) is substantially restricted with respect to the bone; and
a plurality of guiding elements (304) aligning with a cut plane and configured to guide placement of a plurality of reference pins in the bone and on the cut plane; and
ii) a second guide (400), separate from the first guide, the second guide comprising:
a plurality of apertures ( 404) each with a shape corresponding to a reference pin, each of the plurality of apertures (404) configured to receive one of the plurality of reference pins upon sliding the second guide ( 400) over the reference pins after removal of the first guide (300) and substantially restrict movement of the second guide (400) with respect to the bone when the plurality of reference pins are received; and
at least one functional element (402A, 402B) for drilling a hole in the bone,
wherein the method comprises:
a) designing the patient-specific system using the method of any preceding claim; and
b) manufacturing the patient-specific system based on the design resulting from step a) using an additive manufacturing technique.

14. A system for manufacturing a patient-specific system for performing an osteotomy on a bone of a patient, the patient-specific system comprising:
i) a first guide (300) comprising:
at least one contact surface configured to conform to at least one portion of the bone such that when the at least one contact surface is positioned on the at least one portion of the bone, movement of the first guide (300) is substantially restricted with respect to the bone; and
a plurality of guiding elements (304) aligning with a cut plane and configured to guide placement of a plurality of reference pins in the bone and on the cut plane; and
ii) a second guide (400), separate from the first guide, the second guide comprising:
a plurality of apertures ( 404) each with a shape corresponding to a reference pin, each of the plurality of apertures (404) configured to receive one of the plurality of reference pins upon sliding the second guide ( 400) over the reference pins after removal of the first guide (300) and substantially restrict movement of the second guide (400) with respect to the bone when the plurality of reference pins are received; and
at least one functional element (402A, 402B) for drilling a hole in the bone,
wherein the system for manufacturing the patient-specific system comprises:
a) a computing device configured for designing the patient-specific system using the method of any one of claims 1-13; and
b) an additive manufacturing device directly or indirectly connected to the computing device, for manufacturing the patient-specific system.

## Patentansprüche

1. Verfahren zum Entwerfen eines patientenspezifischen Systems zum Durchführen einer Osteotomie an einem Knochen eines Patienten, das patientenspezifische System umfassend:
i) eine erste Führung (300), umfassend:
mindestens eine Kontaktfläche, die konfiguriert ist, um sich an mindestens einen Abschnitt des Knochens derart anzupassen, dass, wenn die mindestens eine Kontaktfläche auf dem mindestens einen Abschnitt des Knochens positioniert ist, die Bewegung der ersten Führung (300) in Bezug auf den Knochen im Wesentlichen eingeschränkt ist; und
eine Vielzahl von Führungselementen (304), die mit einer Schnittebene ausgerichtet sind und konfiguriert sind, um die Platzierung einer Vielzahl von Referenzstiften im Knochen und auf der Schnittebene zu leiten; und
ii) eine zweite Führung (400), die von der ersten Führung getrennt ist, die zweite Führung umfassend:
eine Vielzahl von Öffnungen (404), die jeweils eine Form aufweisen, die einem Referenzstift entspricht, wobei jede der Vielzahl von Öffnungen (404) konfiguriert ist, um einen der Vielzahl von Referenzstiften aufzunehmen, wenn die zweite Führung (400) nach dem Entfernen der ersten Führung (300) über die Referenzstifte geschoben wird, und die Bewegung der zweiten Führung (400) in Bezug auf den Knochen im Wesentlichen einzuschränken, wenn die Vielzahl von Referenzstiften aufgenommen ist; und
mindestens ein Funktionselement (402A, 402B) zum Bohren eines Lochs in den Knochen,
wobei das Verfahren einen computerimplementierten Schritt umfasst:
Bestimmen der Schnittebene gemäß einem Plan, um während der Osteotomie den Knochen des Patienten zu schneiden, indem ein Sägeblatt entlang der Vielzahl von Referenzstiften geschoben wird.

2. Verfahren nach Anspruch 1, wobei die Vielzahl von Referenzstiften eine Vielzahl von Führungsdrähten umfasst, die konfiguriert sind, um das Sägeblatt zum Durchführen der Osteotomie führen.

3. Verfahren nach Anspruch 1, wobei die Vielzahl von Führungselementen (304) eine Bohrführung umfasst.

4. Verfahren nach Anspruch 1, wobei die zweite Führung (400) ferner eine erste Kontaktfläche umfasst, die konfiguriert ist, um sich an mindestens einen zweiten Abschnitt des Knochens anzupassen, wenn die Vielzahl von Referenzstiften aufgenommen wird, wobei die erste Kontaktfläche derart konfiguriert ist, um das der Kontakt zwischen der ersten Kontaktfläche und dem Knochen allein nicht ausreicht, um die Bewegung der zweiten Führung (400) in Bezug auf den Knochen im Wesentlichen einzuschränken.

5. Verfahren nach Anspruch 1, wobei das mindestens eine Funktionselement (402A, 402B) eine Bohrführung umfasst.

6. Verfahren nach Anspruch 1, ferner umfassend ein Osteosyntheseimplantat, wobei das Osteosyntheseimplantat optional eine Platte umfasst.

7. Verfahren nach Anspruch 6, wobei das Osteosyntheseimplantat eine erste Öffnung und eine zweite Öffnung umfasst, wobei jede der ersten und zweiten Öffnungen konfiguriert ist, um ein Implantatfixierungselement aufzunehmen, wobei das mindestens eine Funktionselement eine Vielzahl von Bohrführungen umfasst, und wobei die erste Öffnung und die zweite Öffnung jeweils konfiguriert sind, um sich mit einer Position auf dem Knochen auszurichten, die einer Position auf dem Knochen einer der Vielzahl von Bohrführungen entspricht, wenn die Vielzahl von Referenzstiften von den mehreren Öffnungen aufgenommen wird.

8. Verfahren nach Anspruch 7, wobei:
die erste Öffnung und die zweite Öffnung durch einen ersten Abstand voneinander getrennt sind, und wobei eine erste Bohrführung, die der ersten Öffnung entspricht, und eine zweite Bohrführung, die der zweiten Öffnung entspricht, durch einen zweiten Abstand voneinander getrennt sind, wobei der erste Abstand sich vom zweiten Abstand unterscheidet, oder
wobei die erste Öffnung einer ersten Knochenposition zugeordnet ist, die sich auf einer ersten Seite der Schnittebene befindet, und wobei die zweite Öffnung einer zweiten Knochenposition zugeordnet ist, die sich auf einer zweiten Seite der Schnittebene befindet, wobei die erste Seite der zweiten Seite gegenüberliegt.

9. Verfahren nach Anspruch 1, wobei das mindestens eine Funktionselement eine Schnittführungsfläche und/oder ein oder mehrere röntgendichte Elemente umfasst.

10. Verfahren nach Anspruch 1, wobei die erste Führung (300) abgeschrägte Kanten aufweist und/oder die erste Führung (300) einem Profil zwischen 1 und 3 mm entspricht.

11. Verfahren nach Anspruch 1, wobei sich die zweite Führung (400) nicht mehr als 3 mm über die Vielzahl von Öffnungen (404) und das mindestens eine Funktionselement hinaus erstreckt.

12. Verfahren nach Anspruch 1, wobei die erste Führung (300) flexible Abschnitte zum Entfernen der ersten Führung (300) aus dem Knochen umfasst, wobei die Referenzstifte in der Vielzahl von Führungselementen (304) platziert sind.

13. Verfahren zur Herstellung eines patientenspezifischen Systems zur Durchführung einer Osteotomie an einem Knochen eines Patienten, das patientenspezifische System, umfassend:
i) eine erste Führung (300), umfassend:
mindestens eine Kontaktfläche, die konfiguriert ist, um sich an mindestens einen Abschnitt des Knochens derart anzupassen, dass, wenn die mindestens eine Kontaktfläche auf dem mindestens einen Abschnitt des Knochens positioniert ist, die Bewegung der ersten Führung (300) in Bezug auf den Knochen im Wesentlichen eingeschränkt ist; und
eine Vielzahl von Führungselementen (304), die mit einer Schnittebene ausgerichtet sind und konfiguriert sind, um die Platzierung einer Vielzahl von Referenzstiften im Knochen und auf der Schnittebene zu leiten; und
ii) eine zweite Führung (400), die von der ersten Führung getrennt ist, die zweite Führung umfassend: eine Vielzahl von Öffnungen (404), die jeweils eine Form aufweisen, die einem Referenzstift entspricht, wobei jede der Vielzahl von Öffnungen (404) konfiguriert ist, um einen der Vielzahl von Referenzstiften aufzunehmen, wenn die zweite Führung (400) nach dem Entfernen der ersten Führung (300) über die Referenzstifte geschoben wird, und die Bewegung der zweiten Führung (400) in Bezug auf den Knochen im Wesentlichen einschränkt, wenn die Vielzahl von Referenzstiften aufgenommen ist; und
mindestens ein Funktionselement (402A, 402B) zum Bohren eines Lochs in den Knochen,
wobei das Verfahren umfasst:
a) Entwerfen des patientenspezifischen Systems unter Verwendung des Verfahrens nach einem der vorstehenden Ansprüche; und
b) Herstellen des patientenspezifischen Systems auf Grundlage des aus Schritt a) resultierenden Designs unter Verwendung einer additiven Fertigungstechnik.

14. System zur Herstellung eines patientenspezifischen Systems zur Durchführung einer Osteotomie an einem Knochen eines Patienten, das patientenspezifische System umfassend:
i) eine erste Führung (300), umfassend:
mindestens eine Kontaktfläche, die konfiguriert ist, um sich an mindestens einen Abschnitt des Knochens derart anzupassen, dass, wenn die mindestens eine Kontaktfläche auf dem mindestens einen Abschnitt des Knochens positioniert ist, die Bewegung der ersten Führung (300) in Bezug auf den Knochen im Wesentlichen eingeschränkt ist; und
eine Vielzahl von Führungselementen (304), die mit einer Schnittebene ausgerichtet sind und konfiguriert sind, um die Platzierung einer Vielzahl von Referenzstiften im Knochen und auf der Schnittebene zu leiten; und
ii) eine zweite Führung (400), die von der ersten Führung getrennt ist, die zweite Führung umfassend:
eine Vielzahl von Öffnungen (404), die jeweils eine Form aufweisen, die einem Referenzstift entspricht, wobei jede der Vielzahl von Öffnungen (404) konfiguriert ist, um einen der Vielzahl von Referenzstiften aufzunehmen, wenn die zweite Führung (400) nach dem Entfernen der ersten Führung (300) über die Referenzstifte geschoben wird, und die Bewegung der zweiten Führung (400) in Bezug auf den Knochen im Wesentlichen einschränkt, wenn die Vielzahl von Referenzstiften aufgenommen ist; und
mindestens ein Funktionselement (402A, 402B) zum Bohren eines Lochs in den Knochen,
wobei das System zur Herstellung des patientenspezifischen Systems umfasst:
a) ein Computergerät, das zum Entwerfen des patientenspezifischen Systems unter Verwendung des Verfahrens nach einem der Ansprüche 1 bis 13 konfiguriert ist; und
b) ein direkt oder indirekt mit dem Computergerät verbundenes Gerät zur additiven Fertigung zur Herstellung des patientenspezifischen Systems.

## Revendications

1. Procédé de conception d'un système spécifique au patient pour réaliser une ostéotomie sur un os d'un patient, le système spécifique au patient comprenant :
i) un premier guide (300) comprenant :
au moins une surface de contact conçue pour se conformer à au moins une partie de l'os, de telle sorte que lorsque l'au moins une surface de contact est positionnée sur l'au moins une partie de l'os, le mouvement du premier guide (300) est sensiblement restreint par rapport à l'os ; et
une pluralité d'éléments de guidage (304) alignés sur un plan de coupe et conçus pour guider le placement d'une pluralité de broches de référence dans l'os et sur le plan de coupe ; et
ii) un second guide (400), séparé du premier guide, le second guide comprenant :
une pluralité d'ouvertures (404) ayant chacune une forme correspondant à une broche de référence, chacune de la pluralité d'ouvertures (404) étant conçue pour recevoir l'une de la pluralité de broches de référence lors du glissement du second guide (400) sur les broches de référence après le retrait du premier guide (300) et pour restreindre sensiblement le mouvement du second guide (400) par rapport à l'os lorsque la pluralité de broches de référence est reçue ; et
au moins un élément fonctionnel (402A, 402B) pour percer un trou dans l'os,
dans lequel le procédé comprend une étape mise en oeuvre par ordinateur consistant à :
déterminer le plan de coupe conformément à un plan visant, pendant l'ostéotomie, à couper l'os du patient en faisant glisser une lame de scie contre la pluralité de broches de référence.

2. Procédé selon la revendication 1, dans lequel la pluralité de broches de référence comprend une pluralité de fils-guides conçus pour guider la lame de scie lors de la réalisation de l'ostéotomie.

3. Procédé selon la revendication 1, dans lequel la pluralité d'éléments de guidage (304) comprend un guide-foret.

4. Procédé selon la revendication 1, dans lequel le second guide (400) comprend en outre une première surface de contact conçue pour se conformer à au moins une seconde partie de l'os lorsque la pluralité de broches de référence est reçue, dans lequel la première surface de contact est conçue de telle sorte que le contact entre la première surface de contact et l'os seul est insuffisant pour restreindre sensiblement le mouvement du second guide (400) par rapport à l'os.

5. Procédé selon la revendication 1, dans lequel l'au moins un élément fonctionnel (402A, 402B) comprend un guide-foret.

6. Procédé selon la revendication 1, comprenant en outre un implant d'ostéosynthèse, dans lequel l'implant d'ostéosynthèse comprend éventuellement une plaque.

7. Procédé selon la revendication 6, dans lequel l'implant d'ostéosynthèse comprend une première ouverture et une seconde ouverture, dans lequel la première ouverture et la seconde ouverture sont chacune conçues pour recevoir un élément de fixation d'implant, dans lequel l'au moins un élément fonctionnel comprend une pluralité de guides-forets, et dans lequel la première ouverture et la seconde ouverture sont chacune conçues pour s'aligner sur une position sur l'os correspondant à une position sur l'os de l'un de la pluralité de guides-forets lorsque la pluralité de broches de référence est reçue par la pluralité d'ouvertures.

8. Procédé selon la revendication 7, dans lequel :
la première ouverture et la seconde ouverture sont séparées par une première distance, et dans lequel un premier guide-foret correspondant à la première ouverture et un second guide-foret correspondant à la seconde ouverture sont séparés par une seconde distance, dans lequel la première distance est différente de la seconde distance, ou
dans lequel la première ouverture est associée à une première position d'os qui est située sur un premier côté du plan de coupe et dans lequel la seconde ouverture est associée à une seconde position d'os qui est située sur un second côté du plan de coupe, le premier côté étant opposé au second côté.

9. Procédé selon la revendication 1, dans lequel l'au moins un élément fonctionnel comprend une surface de guidage de coupe et/ou un ou plusieurs éléments radio-opaques.

10. Procédé selon la revendication 1, dans lequel le premier guide (300) comprend des bords effilés et/ou le premier guide (300) se conforme à un profil compris entre 1 et 3 mm.

11. Procédé selon la revendication 1, dans lequel le second guide (400) ne dépasse pas de plus de 3 mm la pluralité d'ouvertures (404) et l'au moins un élément fonctionnel.

12. Procédé selon la revendication 1, dans lequel le premier guide (300) comprend des parties flexibles pour retirer le premier guide (300) de l'os avec les broches de référence placées dans la pluralité d'éléments de guidage (304).

13. Procédé de fabrication d'un système spécifique à un patient pour réaliser une ostéotomie sur un os d'un patient, le système spécifique au patient comprenant :
i) un premier guide (300) comprenant :
au moins une surface de contact conçue pour se conformer à au moins une partie de l'os, de telle sorte que lorsque l'au moins une surface de contact est positionnée sur l'au moins une partie de l'os, le mouvement du premier guide (300) est sensiblement restreint par rapport à l'os ; et
une pluralité d'éléments de guidage (304) alignés sur un plan de coupe et conçus pour guider le placement d'une pluralité de broches de référence dans l'os et sur le plan de coupe ; et
ii) un second guide (400), séparé du premier guide, le second guide comprenant : une pluralité d'ouvertures (404) ayant chacune une forme correspondant à une broche de référence, chacune de la pluralité d'ouvertures (404) étant conçue pour recevoir l'une de la pluralité de broches de référence lors du glissement du second guide (400) sur les broches de référence après le retrait du premier guide (300) et restreindre sensiblement le mouvement du second guide (400) par rapport à l'os lorsque la pluralité de broches de référence est reçue ; et
au moins un élément fonctionnel (402A, 402B) pour percer un trou dans l'os,
dans lequel le procédé comprend :
a) la conception du système spécifique au patient à l'aide du procédé selon l'une quelconque revendication précédente ; et
b) la fabrication du système spécifique au patient sur la base de la conception résultant de l'étape a) à l'aide d'une technique de fabrication additive.

14. Système de fabrication d'un système spécifique au patient pour réaliser une ostéotomie sur un os d'un patient, le système spécifique au patient comprenant :
i) un premier guide (300) comprenant :
au moins une surface de contact conçue pour se conformer à au moins une partie de l'os, de telle sorte que lorsque l'au moins une surface de contact est positionnée sur l'au moins une partie de l'os, le mouvement du premier guide (300) est sensiblement restreint par rapport à l'os ; et
une pluralité d'éléments de guidage (304) alignés sur un plan de coupe et conçus pour guider le placement d'une pluralité de broches de référence dans l'os et sur le plan de coupe ; et
ii) un second guide (400), séparé du premier guide, le second guide comprenant :
une pluralité d'ouvertures (404) ayant chacune une forme correspondant à une broche de référence, chacune de la pluralité d'ouvertures (404) étant conçue pour recevoir l'une de la pluralité de broches de référence lors du glissement du second guide (400) sur les broches de référence après le retrait du premier guide (300) et pour restreindre sensiblement le mouvement du second guide (400) par rapport à l'os lorsque la pluralité de broches de référence est reçue ; et
au moins un élément fonctionnel (402A, 402B) pour percer un trou dans l'os,
dans lequel le système de fabrication du système spécifique au patient comprend :
a) un dispositif informatique configuré pour concevoir le système spécifique au patient à l'aide du procédé selon l'une quelconque des revendications 1 à 13 ; et
b) un dispositif de fabrication additive directement ou indirectement connecté au dispositif informatique, pour fabriquer le système spécifique au patient.
